# EUROPEAN PATENT APPLICATION

(11) **EP 3 379 250 A1**
(43) Date of publication of application: **26.09.2018**
(21) Application number: 17161825.9
(22) Date of filing: 20.03.2017
(51) Int. Cl.: G01N 33/543, C12Q 1/68

(54) **INDICATOR RELEASE SYSTEM FOR THE DETECTION OF AN ANALYTE IN A FOODSTUFF, TEST STRIP THEREFOR, AND ANALYSIS METHOD**

(71) Applicant: Bundesrepublik Deutschland, vertreten durch den Bundesminister für Wirtschaft und Energie, 12205 Berlin (DE); FOSS Analytical A/S, 3400 Hillerød (DK)
(72) Inventor: Rurack, Knut, 12101 Berlin (DE); Climent, Estela, 12459 Berlin (DE); Gil, Tamir, 3400 Hilleroed (DK); Holm, Claus, 3400 Hilleroed (DK)
(74) Representative: Zimmermann & Partner Patentanwälte mbB

(57) **Abstract**

An indicator reservoir, comprising a porous support material and an indicator substance which is enclosed in pores of the porous support material by a releasable pore-closing material, the pore closing material being bound to the porous support material by a compound that is anchored to the porous support material, the indicator substance being released from the pores when the pore-closing material binds an analyte being present in a liquid that wets the indicator reservoir.

## Description

The invention relates to the field of rapid tests for monitoring the compliance with limit values in foodstuffs. Said invention relates especially to the detection of antibiotics, of mycotoxins and hormonally active substances in a liquid agricultural sample and in liquid foodstuffs, such as e.g. milk.

Owing to industrial agriculture and to the global, anthropogenic impact on the environment, liquid foodstuffs, such as milk for example, may contain harmful substances such as antibiotics, mycotoxins or hormonally active substances (e.g. hormones and analogues). The highest maximum quantities for residues of, for example, antibiotics and aflatoxins in milk are set by the legislature to very low concentrations within the range from ng/kg to µg/kg. Specifically in the case of foodstuffs such as milk, which are collected on-the-spot from individual producers, it is therefore critical to identify contaminated batches preferably prior to collection and to thus avoid a contamination of, for example, a relatively large tank load already with the producer or with the farmer, i.e. on-the-spot. For such scenarios, a sensitive analysis is required, in which case the collection of sample, the transport of the sample to a laboratory and an examination which can only be carried out in said laboratory would be impractical and much too expensive for a comprehensive monitoring of the compliance with maximum quantities to be possible at all.

There is therefore a need for novel analytical chemical detection methods which are very simple, relatively rapid and robust, which can be used on-the-spot by the unskilled user and, at the same time, still work with sufficient sensitivity, reliability and accuracy. Nevertheless, even laboratories of different sizes could benefit from a method with these aforementioned properties, as it may make them more efficient and cost-effective.

Against this background, there is proposed, according to one embodiment, an indicator reservoir, comprising a porous support material having pores and an indicator substance which is contained in the pores of the porous support material by a releasable pore-closing material. The organic pore closing material is bound disengageably (i.e. non-covalently) to the support material. In particular, the pore-closing material is non-covalently bound to the porous support material by a compound that is anchored to the porous support material. The pore-closing material is selected to specifically bind an analyte. The indicator substance is released from the pores when the pore-closing material binds the analyte. The analyte is typically contained (or not contained) in a liquid that wets the indicator reservoir. In other words, all pores at the surface of the porous support material are capped, and thus closed, by the pore closing material. Therefore, the indicator substance which is stored inside the pores cannot be released into a surrounding liquid, as long as the analyte is not specifically bound by the pore-closing material and the pore-closing material released from the porous support material. If a liquid which contains the analyte comes into contact with the indicator reservoir that allows the non-covalent bonds to be detached and enables specific bond(s) between the pore-closing material and the analyte to be formed and thus prevents reformation of the non-covalent bond between the pore-closing material and the anchored compound. If the wetting liquid does not contain the analyte, the disengageable (i.e. non-covalent) bond will be strong enough to hold the pore closing material in the proximity of the pore. Therefore, the pores are blocked and the indicator substance is thus substantially prevented from escaping the pores and hence, no signal is generated.

The liquid which needs to be characterized with respect to a content of the analyte is typically an aqueous solution or suspension. The liquid may be selected from: a liquid foodstuff, a liquid agricultural sample, or a liquid that was used to extract the analyte from other type of foodstuff or agriculture-related substance.

It is noted that, as used herein and in the appended claims, a liquid foodstuff in the sense of the present application comprises liquid groceries like bottled drinking water, juices, milk and liquid dairy products, soups, sauces, dressings, liquid convenience foods, alcoholic and non-alcoholic beverages like wine, beer, cider, liquid condiments such as soy, vinegar, a honey, an egg or a constituent thereof, an edible oil or any other perishable liquid food.

It is noted that, as used herein and in the appended claims, a liquid agricultural sample is understood to encompass any liquid selected from a process water or wastewater of immediate agricultural origin, i.e. drainage water of arable or pasturable land, an irrigation water or a water for or from processing of a harvested crop, a water coming from drainage of animal farms and/or of cultivated soils, a physiological fluid that is taken from livestock animals to monitor their health and wellbeing, selected from a blood, a blood component, an urine or an extracellular fluid sample.

However, such physiological liquids like blood, serum, urine, saliva or fractions thereof which have been taken from a pet like, e.g., a cat, a dog, a canary, common pet parakeet or other house pets are excluded from the understanding of a liquid agricultural sample. Any samples taken from a human being, e.g. from an agricultural worker, are also not considered to be agricultural samples. Samples taken from tap water are also not considered as agricultural samples or foodstuff. Tap water is considered to comprise a non-bottled drinking water delivered for direct use, e.g. in a household. General environmental samples taken from a water body or from a soil which is not under agricultural use are also not considered to comprise agricultural samples.

Furthermore, non-agricultural surface water, sea water and water of lakes and rivers as well as ground water, industrial waste water, and household waste water are not considered to comprise a liquid foodstuff or to comprise a liquid agricultural sample or agriculture related samples. Such water and waste water is explicitly excluded from the understanding of a liquid agricultural sample as used in the present description and claims.

It is noted that, as used herein and in the appended claims, an agriculture-related substance is understood to mean a substance which is directly agriculture-related such as a fertilizer, a pesticide such as a fungicide, an acaricide, an insecticide, an herbicide, a rodenticide or a growth regulator; a seed treatment or a seed dressing material.

The pores of the e.g. inorganic porous support material are closed by an entity which comprises an organic molecule (hapten-like substance) which is associated with a pore closing material. The pore closing material is non-covalently bound to an organic molecule (referred to therein as compound) which is tightly fixed at the surface of the support material. The indicator substance referred to - initially arranged within the pores - is releasable from the pores, by detaching of the pore closing material from the pore openings when the pore-closing material binds a particular analyte in foodstuff - i.e. one assigned to the specific indicator substance - which is present in a liquid foodstuff. After binding to the analyte the pore closing material is hindered to rebind to the organic molecule (which is e.g. a hapten-like substance) which is fixed at the support and thus the pore-closing material is detached.

The pore closing material is selected from the group comprising an antibody, a receptor protein, and an aptamer. The aptamer may be selected from an oligo-aptamer and a peptide aptamer. The antibody, the receptor protein, and the aptamer are able to specifically bind the analyte.

The following description of the basic mechanism of the suggested indicator reservoir and of the corresponding indicator release system may be given, for example, if the pore closing material comprises an antibody:

The bond(s) between an antibody and a hapten or the bond(s) between an antibody and an organic molecule which resembles a hapten derivative is(are) non-covalent.

Therefore, the pore closing cap (e.g. the antibody) associates and dissociates all the time. The resulting system is dynamic. Since in such dynamic systems the association occurs very fast, whereas the dissociation is rather slow, only insignificant leaching of the indicator substance from the pores of the indicator reservoir occurs.

Furthermore, the affinity of the analyte towards the antibody is much higher in comparison to the affinity of the hapten towards the antibody. Therefore, the binding site of the antibody (cap) is effectively blocked in presence of the analyte. Hence, any fast rebinding of the antibody to the hapten is prevented.

Therefore, the antibody may depart from the pore opening sufficiently far while its binding site is blocked.

Therefore, even in the case of a dissociation of the complex between the analyte and the antibody - which comprises a dynamic system as well - no resealing of the pore(s) may occur.

As to the mentioned above affinities, the affinity of the antibody towards the analyte is much higher in comparison to the affinity of the antibody towards the hapten as well.

In this connection, a foodstuff contaminant which is regarded as a typical analyte is understood to mean such a chemical substance, the presence of which must be monitored for the purpose of non-exceedance of an officially defined maximum concentration. Foodstuff contaminants are thus substances which neither occur naturally in the customary raw product, nor are added during the customary foodstuff manufacturing process. More particularly, they are contaminants from the environment or in the course of agricultural cultivation, animal husbandry or the manufacturing process which reach the raw product or the foodstuff itself. Typical examples are pesticides, veterinary medicaments and animal feed substances (e.g. mycotoxins, substances having hormonal activity) that cross over to plant or animal products or toxins from moulds (mycotoxins) on infested raw product.

According to an embodiment the pore-closing material comprises an antibody, an antibody fragment, a receptor protein, or an aptamer. Advantageously such molecules are able to specifically bind different types of analytes.

According to an embodiment in the indicator reservoir the compound that is anchored to the support material comprises an organic molecule. In particular, it comprises a hapten, a hapten-like organic molecule, a complex of a metal ion with (an) organic ligand(s), a nucleic acid or its strand fragment, or any epitope or peptide molecule. Such molecules are known to be specifically bound by specific antibodies, their fragments, by specific aptamers, and by enzymes.

The binding of the antibody or of the hapten is effected by, e.g., a liquid foodstuff (e.g. milk) wetting the indicator reservoir, i.e. as a result of a contact of the reservoir with a wetting liquid foodstuff. The type and/or quantity of the indicator substance released in the presence of the analyte indicates the presence of the analyte in the liquid foodstuff in a qualitative manner and/or at least semi-quantitative manner, if not in a completely quantitative manner, within a pre-determinable concentration range relevant to the practice of foodstuff monitoring.

The advantages of said embodiment consist in the free use of the indicator reservoir, either on a test strip or in the free solution, for example in a cuvette or in a microfluidic system. Such can be used for on-the-spot monitoring of the foodstuff-chemical in liquid foodstuffs. For such purpose the indicator reservoir may be applied to an appropriate substrate (e.g. a paper strip or a porous test stick), or may be introduced into a cuvette or into a microfluidic system which contains the liquid sample. The test strip, the cuvette or the microfluidic system are then measured in a spectroscopic, electrochemical, colorimetric or other indicator-specific manner. A substantial advantage of using a receptor protein arises from the possibility of setting up generic assays, since, for example, the use of a carboxypeptidase would allow the detection of a group of beta-lactam antibiotics, since these proteins recognize the active form of the beta-lactam ring structure (cf., for example, Biacore Journal - Number 2 2003, 22-23).

According to one embodiment, the pore closing material has been adapted and/or selected with respect to its specificity and/or affinity such that it specifically binds to an analyte in foodstuff, e.g. to a mycotoxin, to an antibiotic or to a substance acting as a hormone or hormone analogue.

The advantages of said embodiment consist in the detection event - the binding of the analyte by an antibody, a receptor protein or an aptamer and the thus associated opening of the pores of the reservoir - being decoupled from the signal generation, i.e. from the measurement of the released indicators. For instance, an individual binding event can release a multiplicity of indicators, amounting to a signal amplification and thus a higher sensitivity. Furthermore, such a system has a high degree of modularity in the design.

According to one embodiment, the pore closing material is adapted to specifically bind an analyte substance or a specific group of analyte substances selected from: a toxin, an antibiotic, a hormone or a hormonally-effective substance, an allergen, a digestion deficiency causing substance, a transmissible spongiform, i.e. a prion, a genetically modified organism or a constituent thereof, an adulterant, a nutrition additive, or an enzyme.

Therein, in the case of digestion deficiency causing substances the target substances are biochemicals needed by the organism to breakdown, assimilate, and eliminate nutrients. These include:
- Digestive enzymes such as protease, amylase, lipase, cellulase, sucrase or lactase;
- Regulatory digestive hormones, such as gastrin (stomach), secretin (small intestine), cholecytokinin (small intestine), gastric inhibitory peptide (small intestine), and motilin (small intestine);
- Stomach acid and bile acids.

Antibiotics and mycotoxins are those contaminants of all sorts of liquid food, e.g. milk, that have the greatest practical significance. The reliable detection of substitutes like adulterants is of great significance from a practical point of view as well. Pathogens like prions, toxins, and allergens need to be excluded from food for obvious reasons. Furthermore, the content of other substances must be known in order to be precisely indicated.

According to an embodiment the pore-closing material is adapted to specifically bind to an analyte micro-organism or to a specific group of analyte micro-organisms selected from: a bacteria, a bacteria spore, a pathogen, a mold, a yeast, a protozoon, a zoonosis causing agent, or a virus. Advantageously intoxications can be omitted and safe food can be provided.

According to an embodiment the pore-closing material is adapted or selected by e.g. affinity chromatography purification or as a monoclonal antibody to specifically bind to an analyte which comprises a specific nucleotide sequence or a group of specific nucleotide sequences.

According to one embodiment, the antibody is an antibody fragment, for example a Fab or a F(ab')₂. Moreover, the receptor protein is a protein having a particular fit for small molecules or for parts of larger molecules which bind to the receptor structure, e.g. penicillin-binding proteins (PBP) as specific receptor proteins. PBP are bacterial enzymes which are involved in cell wall synthesis and represent a point of attack for β-lactam antibiotics. They are capable of specifically binding β-lactam antibiotics.

The advantages of said embodiment consist in the relatively high specificity between the antibodies or receptor proteins and the small molecules, e.g. a mycotoxin, an antibiotic, an adulterant, or a hormonally active substance.

According to one embodiment, the porous support material encompasses a mesoporous material. The mesoporous material may comprise, e.g., a SiO₂ particle having mesopores, wherein mesopores have an average pore diameter of between 2 nm and 50 nm.

The advantages of said embodiment consist in the high loading capacity of the mesoporous porous support particles for indicator molecules. The mesoporous SiO₂ particles have an ordered three-dimensional structure which easily allows the transport of indicator molecules from the reservoir into the surrounding solution.

According to one embodiment, the aforementioned mesoporous material comprises one of SiO₂ and Al₂O₃. It may be selected from a nanoparticle or a microparticle. Nanoparticles have arithmetically averaged outer diameters for a substantially spherical shape within a range from 40 to 500 nm, typically within a range between 80 nm and 130 nm. Microparticles have arithmetically averaged outer diameters within a range from 0.5 µm to 200 µm, typically within a range between 0.8) µm and 5 µm.

The advantages of said embodiment consist in the control of the particle size and thus the quantity of indicators with which the particles can be loaded.

According to one embodiment the supporting porous material comprises a nanoporous structure which comprises a magnetic or a paramagnetic material, e.g. a magnetic or paramagnetic particle. Advantageously such materials facilitate the effective separation of the indicator reservoir from a liquid phase and hence, are suitable for different assay formats. The mesoporous material may, in other embodiments, be comprised of an inorganic material, e.g. one or more of porous carbon, silicon, silicon carbide, silicon oxycarbide, Si carbonitride, Si oxynitride or Si nitride or alumina (Al₂O₃).

According to one embodiment, the indicator substance referred to above is selected from a fluorescent material, from a chemiluminescent material, from an electrochemically active substance and/or from a material having a specific RAMAN spectrum or a specific IR spectrum, within a wavelength range from 10⁻⁸ to 10⁻⁴ m, and a utilized measurement frequency from 10¹⁶ to 10¹² Hz for excitation and detection. This means that the capture of absorption, of luminescence (fluorescence, phosphorescence, bioluminescence, chemiluminescence and electrochemiluminescence) and of Raman scattering may be included for the proposed measurements in order to be able to provide qualitative and/or at least semi-quantitative information about the type and/or concentration of the analytes in question in the liquid sample in question.

Specific advantages of said embodiment consist in the range of applications through the selection of suitable detection methods, for example for transparent, coloured or turbid samples, and the desired embodiments of the support system, of the concentration range to be captured and of the possibility of multiplex detection.

According to one embodiment, the hapten, which together with the antibody, the receptor protein or the aptamer forms the pore-closing compound, is covalently bound to the support material. The chemical nature of the covalent bond can, for example, encompass a Si-O-Si bond. Furthermore, the compound can be bound non-covalently to the support material or can be adsorbed.

Specific advantages of said embodiment consist in the simple functionalization of the mesoporous SiO₂ material, which has silanol groups on the surface and can be easily reacted with organically modified silanes, which are commercially available in great variety. The support particles can thus be chemically adapted in an ideal manner to the analyte, the detection reaction, the sample matrix or the type of application. Adsorption and non-covalent binding are also effective means for surface modification of the porous carrier particles.

According to one embodiment, the support material bears an organic molecule which acts as a hapten for the antibody or for the receptor protein, or as a binding site for the aptamer i.e. for the pore closing material. Said organic molecule is structurally very similar to that organic compound which serves to generate an immune response in the antibody-producing organism (chicken, mouse, rat, rabbit, sheep, goat, donkey, horse) or an antibody-producing cell (e.g. a permanent mouse cell line, or a different hybridoma cell line).

Here, a specificity of the antibody, of the oligo-aptamer, of the peptide aptamer or of the receptor protein, i.e. of the pore-closing material with respect to the analyte is higher than a bond with respect to said organic molecule. Via the selection of functional groups, the specificity and thus the binding constant can be set in a controlled and targeted manner and according to desire as described here: strong bond in relation to the analyte, weaker bond between the compound and the pore closing material (cap, antibody, antibody fragment, aptamer, receptor protein).

Specific advantages of said embodiment consist in an avoidance of non-specific interactions and bonds between antibody, receptor protein or aptamer and support-bound hapten with, additionally, an easily possible bond between antibody, receptor protein or aptamer, on the one hand, and the analyte, on the other, which then leads to a displacement of the antibody, receptor protein or aptamer from the pore opening as explained above.

According to a further embodiment, the support material comprises:
- a periodic mesoporous silica having mesopores between 2-50 nm, for example between 2-20 nm, more particularly between 2-5 nm; and
- a specific surface within a range from 400 to 2000 m²g⁻¹, for example within the range from 500 up to 1500 m²g⁻¹, more particularly within the range between 600 up to 1200 m²g⁻¹.

Specific advantages of said embodiment are the possibilities of adapting the pore size specifically to the size of the biomolecule closing said pore size, of storing the desired quantity of indicator molecules in the reservoir and of adapting diffusion times optimally to the size of the indicator molecules. A large specific surface of the support material moreover provides a sufficient area for binding haptens or other organic molecules.

According to an embodiment the liquid foodstuff is selected from: a water, a milk, a wine, a non-alcoholic beverage, a beer, a cider, a honey, an egg or a constituent thereof, an edible oil, or a waste water from the food industry.

Advantageously, such liquid food and process liquids need to be monitored. The suggested materials and method allow monitoring.

According to an embodiment the liquid agricultural sample is selected from a process water used for agricultural purposes, or a waste water coming from drainage of animal farms and/or directly of cultivated soils. Furthermore, the liquid agricultural sample is selected from a blood or a blood component, a urine from a farmed animal (livestock), or a serum that are taken from a farmed animal (livestock) to monitor its health and well-being.

According to an embodiment the liquid is a liquid that was used to extract an analyte or analytes from non-liquid substances such as grain, rice, corn maize, coffee beans and seeds of all kinds, meat, fish and sea food, plants and vegetables that are used as food and/or animal feed, milk powder and non-liquid milk products, flour and meals (products of a milling process) of all kinds.

Advantageously, contaminants of such materials can be monitored effectively.

According to an embodiment a system for monitoring analytes in liquid foodstuff, in liquid agricultural samples, or in liquid containing extracts from a foodstuff or an agriculture-related substance is suggested which comprises: the indicator reservoir described above, a means for wetting or mixing the said reservoir with the said liquid in order to allow the release of said indicator substance in accordance with a concentration of the analyte in the liquid, a means to separate the indicator reservoir from the liquid containing the released indicator, and a means to detect and to quantify the concentration of the released indicator in the liquid.

Advantageously, the suggested system is adapted to monitoring analytes in liquid food and related matrices in agriculture, animal farming, horticulture, and food technology.

According to an embodiment the system comprises a plurality of different indicator reservoirs, each of the pluralities allowing the specific detection of a different analyte.

Advantageously, different analytes can be detected simultaneously even within one assay.

According to an embodiment a difference among the different indicator reservoirs lies in the spectral, the electrochemical and/or the plasmonic resonance characteristics of the released substance, i.e. the indicator.

Advantageously, different analytes can be detected with different assay formats simultaneously. Even more advantageously, some analytes can be detected simultaneously by different assay formats.

According to an embodiment the means of detection comprise an illumination source and an optical detector that are coupled to an optical filter array, a photo-spectrometer, a galvanometer, and/or a potentiostat.

According to an embodiment the detecting means:
- for an optical detection of analytes in foodstuff comprise an illumination source and an optical detector that are coupled to an optical filter array and a spectrophotometer, optionally incorporating a waveguide or an optical fiber;
   Therein, the name spectrophotometer is considered to encompass any apparatus which is adapted to detect and/or to measure a luminescence, a fluorescence, an optical absorption and/or an optical transmission.
- for an electrochemical detection of analytes comprise a galvanometer and/or a potentiostat which are connected to electrodes; and
- for an opto-electrochemical detection of analytes comprise a galvanometer and/or a potentiostat which are connected to electrodes and also coupled to an optical detector.

According to an embodiment the means of separating the indicator reservoir from the liquid containing the released indicator comprises a magnet or a coil.

According to an embodiment the means of separating the indicator reservoir from the liquid containing the released indicator comprises a centrifuge; a membrane filter; a lateral-flow strip; a liquid phase extraction module; a micro-fluidics separation module; and/or a magnet and magnetic particles, wherein the magnetic particles are functionalized with an agent that binds to the pore-closing material as described above.

According to an embodiment the agent with which the magnetic particles are functionalized comprises an antibody or an antibody fragment, the antibody or antibody fragment comprising a specificity against the pore-closing material.

According to an embodiment said antibody or antibody fragment has a lower affinity to the compound that is binding it to the porous support material and the affinity that said antibody or fragment has to the analyte.

Advantageously, this allows to effectively recognize an analyte and to indicate the presence of the analyte by the released indicator.

According to a further embodiment, the indicator reservoir is arranged on a test strip. A liquid foodstuff wetting the test strip then releases the indicator substance from the pores of the indicator reservoir on the test strip when an analyte contained in the liquid foodstuff is bound by the antibody or by the aptamer owing to the affinity thereof. As a consequence, the detachable/non-covalent bond between the pore-closing material and the hapten (the compound) is disengaged. As a result, the pore-closing material can diffuse away from the porous support material. In this way the pore closing cap, i.e. the pore closing material is removed from the pore opening. As a result, the indicator substance is no longer confined in the pores of the support material. Said indicator substance escapes and can be qualitatively determined with respect to its presence and/or measured, i.e. quantified, with respect to its quantity on a surface of the test strip or in a volume of a liquid. Based on a suitable calibration of the used detecting means, a concentration of the analyte in a sample can be detected.

Specific advantages of said embodiment are the simple implementation of the sensory nanoparticles or microparticles into an easy-to-handle form and the use of imaging readout methods (reading device for lateral flow tests, digital camera, camera of portable communication devices, etc.).

According to another embodiment of the invention, there is proposed a test strip or a test stick which is adapted for a lateral flow assay (LFA). The strip or stick comprises at least one section or one segment provided with an indicator reservoir according to any of the embodiments already described above and yet to be described below in the description text.

Specific advantages of said embodiment are the variety of the usable indicators, the avoidance of cost-intensive antibody/gold nanoparticle conjugates (as in the case of pregnancy tests) and the possibilities in relation to multiplexing. Advantageously, the proposed analysis method and the use of the proposed indicator reservoir does not require antibody/gold conjugates, making it possible to provide the test strips in a cost-effective manner.

According to one embodiment, the test strip comprises a section or segment which is modified by a metal colloid and/or a section or segment which is modified by at least one metallic nanoparticle.

It is known that metal colloids, for example silver nanoparticles and/or nanometer-thick metallic layers in interplay with a RAMAN probe bring about an effect known as SERS: i.e. they increase the sensitivity. As a result of the adsorption of an analyte to the surface of the metal particles or structures, the localized surface plasmons of these nanostructured matrices couple with the Raman shifts in the molecule and lead to an enhancement of the Raman scattering, which usually comprises several orders of magnitude. This yields special advantages for an increased sensitivity of detection of even only the smallest quantities of the sought-for analyte down to the sub-ppm, the ppb range, and ppt range. Such high sensitivity is of practical relevance from a foodstuff-toxicological point of view especially for certain mycotoxins.

According to one embodiment, the proposed test strip comprises a paper or at least a nitrocellulose or glass fiber paper.

Specific advantages of said embodiment are the properties of nitrocellulose of having very good flow properties for rapid assay times, of having a network which efficiently retains nanoparticles and microparticles at the site of deposition, and of not being luminescently or electrochemically active itself.

According to another embodiment, there is proposed an analysis method for detecting a residual quantity of a mycotoxin, of a hormonally effective substance, or of an antibiotic. Said monitoring method comprises the steps of:
- Providing a test strip bearing an indicator reservoir, the indicator reservoir comprising a porous support material and an indicator substance; wherein the indicator substance is enclosed in the pores of the porous support material; wherein the indicator substance is releasable from the pores by a pore-closing entity comprising a pore closing material (cap) and a compound; wherein the indicator substance is releasable from the pores when the pore closing material binds an analyte. The analyte is thus detectable with the aid of the indicator released from the indicator reservoir, wherein the analyte is present in a liquid foodstuff which wets the indicator reservoir;
- Wetting the indicator reservoir with the liquid containing the analyte; and
- Determining, a release of the indicator substance on the test strip, the determination being effected in a qualitative and/or at least semi-quantitative manner.

The advantages of said embodiment concern, e.g., the possibility of a comprehensive on-the-spot monitoring of the batches delivered by small and medium-sized milk producers at a dairy with respect to their content of the substances already referred to above and substances yet to be referred to below (analytes, foodstuff contaminants or foodstuff-contaminating substances).

According to one embodiment, the "wetting" step is selected from the formation of a directed flow of the liquid containing the analyte or at least of one of its liquid components and is achieved with at least partial formation of a front (29) of the same on the test strip (21, 24, 25, 26, 27). In this way, by means of a position of the released indicator substance on the test strip, it is possible to unambiguously determine the presence of the analyte and/or to quantify it with the aid of a tailored reading device.

The advantages of said embodiment arise from the broad acceptance and availability of suitable reading devices, for example: of digital cameras, scanners, intelligent telecommunications terminal devices (e.g.: mobile telephone), readers or similar, single-handedly operable small devices.

According to one embodiment, a measurement device selected for carrying out the described analysis method is selected from: a single-handedly operable reading device or scanner, from a telecommunications terminal device, for example a mobile telephone or "smart phone", or from a digital camera.

The advantages of said embodiment have already been mentioned. These devices are also operable in an easy and error-free manner by the truck driver less familiar with foodstuff analysis or some other assisting person.

The above-described embodiments can be combined with one another as desired. Further embodiments, modifications and improvements are revealed by means of the following description and the attached claims.

According to preferred embodiments, there is thus proposed a test strip assay in conjunction with a portable and rapidly measuring "lateral flow" fluorescence reading device. In this connection, a "lateral flow" fluorescence reading device is understood to mean a reading device which is customarily suitable for the evaluation of fluorescence-based immunochromatographic rapid tests with so-called "lateral flow strips" (LFS) and is typically used outside the laboratory. A substantial advantage of the possible use of the named "lateral flow" fluorescence reading devices consists specifically in the fact that said devices have a robust construction, and work reliably and are usable without any problems even outside a laboratory, for example in the field. This facilitates the analysis of sample material on-the-spot. Owing to the currently attained prevalence of immunochemical LFS tests, the stated fluorescence reading devices are available in a cost-effective manner.

The presently proposed biomolecule-gated indicator release system is capable of responding to the presence of specific analytes with a massive signal amplification because the optical or electrochemical signal is independent of the direct chemical interaction. Since these systems are mesoporous, nanometre-sized hybrid materials and the generated signals can be very easily captured by means of miniaturized detection systems or mobile communication devices. Therefore, it is very easily possible to realize an integration on test strips or in microfluidic chips.

As a result, the capture, or qualitative and quantitative analysis, of the harmful substances referred to becomes rapid, sensitive, specific, inexpensive and simple, this being essential especially for the day-to-day use by unskilled personnel directly on-the-spot or in the field.

Solutions to date for the simple detection of antibiotics or aflatoxins in milk, which detection can also be conducted in the field or "on-the-spot", encompass a range of microbiological (e.g. Delvotest®; DSM Food Specialties, Delft, the Netherlands), chromatographic (e.g. J. Chromatogr. A 1998, 812, 99; J. Chromatogr. A 2003, 987, 227) or immunochemical (Food Control 2011, 22, 993; World Mycotox. J. 2015, 8, 181-191) methods. For example, use is also made of, inter alia, test strips with immunochemical detection (e.g. commercial Aflasensor or Twinsensor detection kit from Unisensor, Belgium).

The basic principle in the case of the test strips consists, for example, of the colorimetric detection via the detection of an aggregation of functionalized gold nanoparticles. Other approaches based on miniaturizable technologies are, for example, electrochemical biosensors, which are integrated into various microfluidic systems (Sensors 2010, 10, 9439), aptamer biosensors (Mater. Sci. Eng. C 2013, 33, 2229) or dynamic light scattering (J. Agric. Food Chem. 2013, 61,4520).

However, the already known solutions are only partly satisfactory and require a high degree of technical effort. For instance, immunochemical, microbiological or chromatographic laboratory methods are typically time-consuming, require expensive instruments and consumable materials and typically cannot be used in on-the-spot detection.

Conventional test strips with immunochemical detection have deficits in accuracy, precision and reproducibility and allow only qualitative Yes/No answers. Moreover, test strips themselves are relatively expensive owing to the use of gold nanoparticles.

It is therefore an object of the present invention to allow a rapid, simple, sensitive and miniaturized detection in order to detect particular organic harmful-substance contamination in liquid foodstuffs such as milk.

The following objects underlie the invention:
Synthesis of hybrid, mesoporous nanoparticles or microparticles containing a chemical indicator substance in the pores, mesoporous particles generally having regularly arranged pores having a diameter between 2 and 50 nm.

The mesoporous particles are covalently functionalized on the outer surface with particular organic molecules (haptens or other organic molecules) similar to the target substances (e.g. antibiotics or aflatoxins). Said haptens or organic molecules have an affinity for a biological receptor molecule (e.g. antibody, receptor protein or aptamers). In addition, a further type of molecules such as oligo- or poly(ethylene glycols) can be attached on the surface in order to increase the binding specificity.

After the loading of the pores and the functionalization of the outer surface of the mesoporous particles, the pore openings are closed with the biochemical macromolecule (e.g. antibody, receptor protein or aptamer).

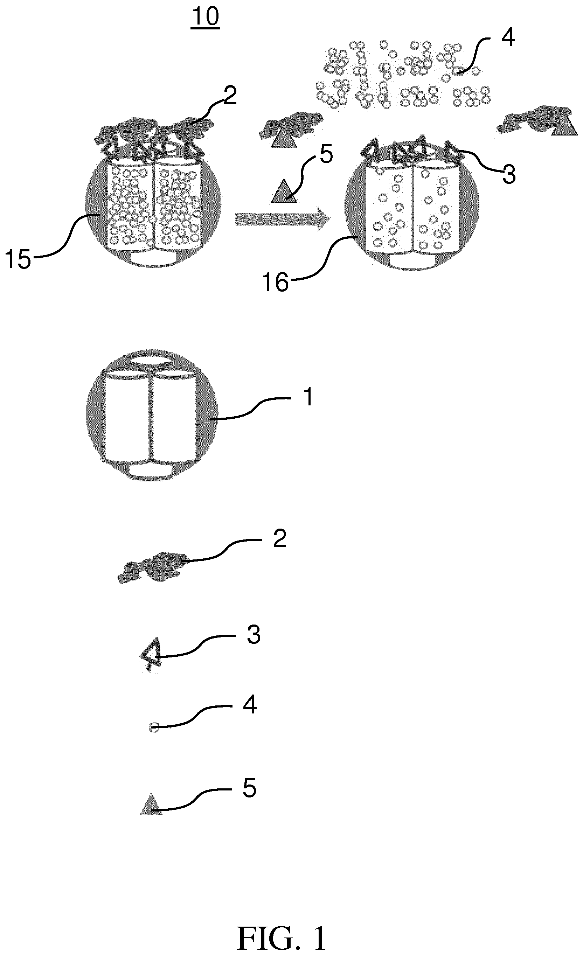

In the presence of the target substance (e.g. antibiotics or aflatoxins) in the liquid foodstuff (e.g. milk) to be examined, the biomolecules blocking the pore openings of the mesoporous material are bound to the particular target substance (e.g. antibiotics or aflatoxins). The pores are thereby opened, the indicator substance subsequently diffuses out and can, for example after spatial separation from the support particles, be measured by means of a particular signal (e.g. optical or electrochemical) (cf. Fig. 1).

In the course of this, one target molecule releases on average a large quantity (typically several hundred) of indicator molecules, and so the system is characterized by a signal amplification. In addition, the substance quantities correlate, making a quantitative analysis possible. In other words, the quantity of the released indicator substance correlates with the concentration of the analyte. Therefore, the indicator signal can be calibrated and an assay based on the indicator reservoir be used for quantitative analysis.

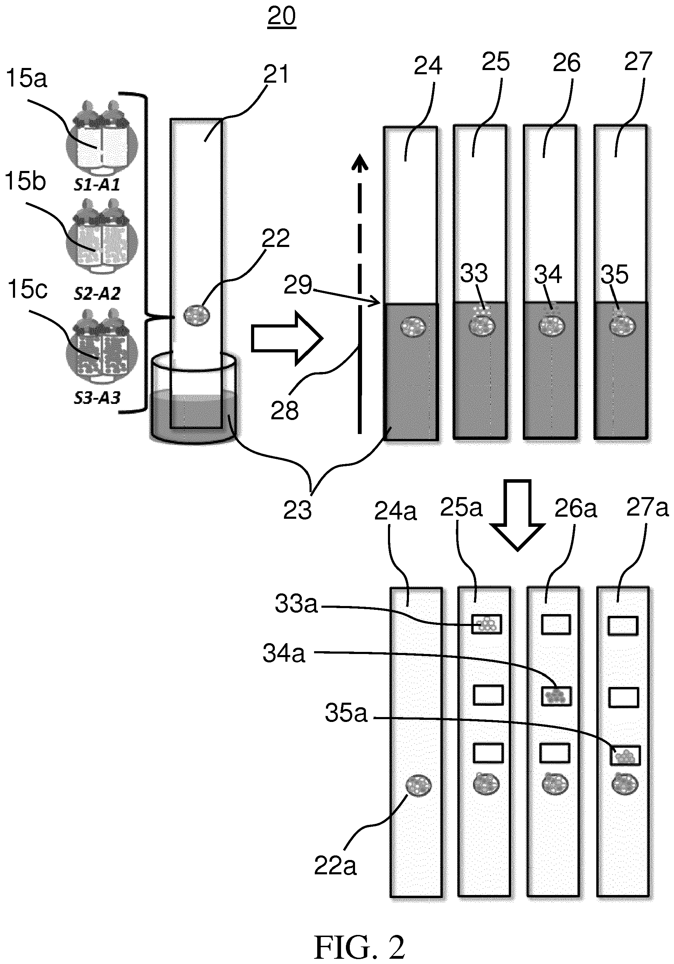

The direct contact of the biomolecules which block the pore openings of the mesoporous material with a liquid food containing the target substance is sufficient for triggering the release reaction. This can, for example, be effected by dipping or dropwise addition using test strips (e.g. Fig. 2) or drop-by-drop addition into a microfluidic system or chip.

The synthesis of the hybrid materials is very flexible because various porous materials, haptens, indicator molecules can be selected. In addition, various biomolecules, especially proteins, can be used for the closure of the pores. This modularity contains an enormous potential not only for on-the-spot analysis, but also for high-throughput screening or for multiplex detection.

An integration of the hybrid, mesoporous sensor materials into, for example, test strips or microfluidic chips allows, at the same time, the development of simple, cost-effective and reproducibly manufacturable rapid tests. Said tests can then be used for, for example, the analytical determination of organic contaminants in milk.

The described object is, for example, achieved by an indicator reservoir according to Claim 1.

The indicator release systems based on nanoparticles or microparticles are usable in miniaturized detection methods in a very diverse manner. Quantitative detection is possible as a result of a spatial separation of support particles and the released indicator, e.g. a released dye.

Since the systems are usable on test strips or in microfluidic chips, liquid foodstuffs can be examined directly on-the-spot for the foodstuff-chemically relevant target substances. The system is very flexible with regard to all components and the eventual detection; it also allows the parallel detection of various parameters through the use of differently loaded and gated particles (multiplexing).

It is therefore possible with surprising ease to conduct a rapid, simple, sensitive and miniaturized detection in order to detect particular organic harmful-substance contamination in liquid foodstuffs such as milk in a quantitative and at least semi-quantitative manner.

What is therefore proposed is a rapid, simple, sensitive and miniaturized detection in order to detect particular organic harmful-substance contamination in liquid foodstuffs such as milk and to determine the content thereof.

The attached drawings illustrate embodiments and serve, together with the description, to elucidate the principles of the invention. The elements of the drawings are relative to one another and not necessarily true to scale. Identical reference signs refer to correspondingly similar parts.
**Figure 1** shows a principle diagram of the course of events for an analysis, viewed microscopically.
**Figure 2** shows a principle diagram of the course of events for the presently proposed analysis of a liquid foodstuff on the basis of 3 examples and one control, viewed macroscopically. Shown diagrammatically is the parallel use of different indicator substances in a mixture of reservoirs of differing specificity, i.e. differing selectivity for other analytes in each case. Mesoporous SiO₂ particles filled with the indicator substances in each case are applied as a mixture in a single spot on the test strip. The arising results of the separation of the different indicator substances for the analytes in question in the liquid foodstuff are depicted diagrammatically in Fig. 2.

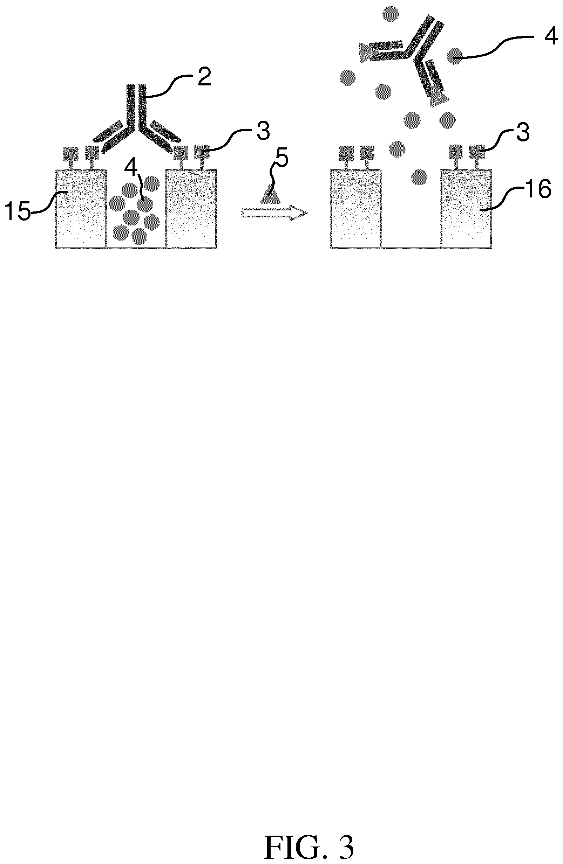

**Figure 3** shows a schematic of analyte-triggered dye release from the mesoporous indicator reservoir according to a practical example which will be described further below. In the gated carrier 15 the pores with trapped inside dye molecules are capped by a modified hapten which is anchored to the surface of the mesoporous particles and carries a capping antibody 2. The antibody has been obtained by immunization of New Zealand white rabbits with a conjugate of bovine serum albumin (BSA) with the analyte sulfothiazole (STZ). Schematically, the analyte 5 is depicted by a green triangle. The anchored epitope derivative 3 is bound by the antibody as long as no analyte 5 is present in solution.

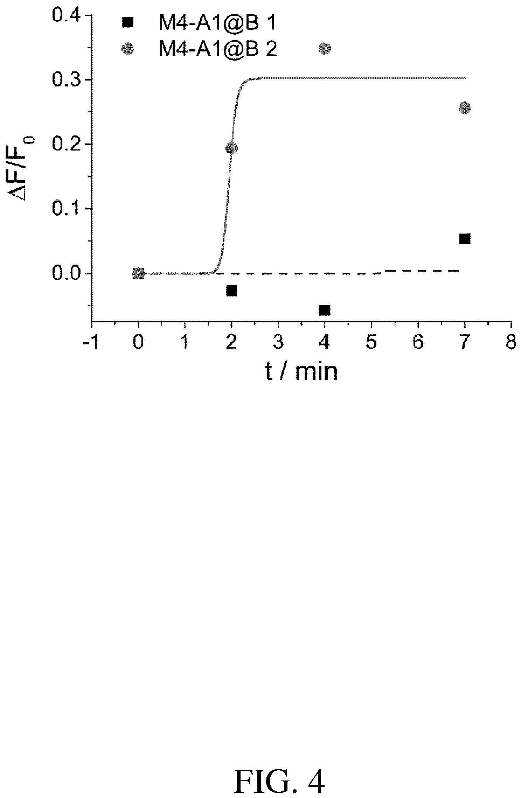

**Figures 4 through 10** illustrate fluorescence characteristics of practical examples as outlined further below.

The test strips can, for example, be gripped at the upper edge with tweezers and dipped into the liquid sample of the foodstuff (e.g. milk) to be examined. In fibrous or porous test strip material, the liquid foodstuff - absorbed by capillary forces - will gradually rise up to the upper edge. In the course of this, the front passes the reservoir (not dipped into the sample). Lateral flow then opens those pores, the pore closing molecules (e.g. antibodies or aptamers) of which react specifically with analyte possibly present in the sample flowing past. In other words, if the analyte makes contact with the pore-closing molecule, the latter is released from the pore rim leaving behind an open pore. The indicator substance intended for the foodstuff analyte in question is immediately released from the opened pores into the laterally flowing liquid phase and migrates behind the front according to chromatographic principles. After completion of the chromatographic separation, i.e. when the front has migrated up to below the upper edge, the test strip is removed from the sample and can be measured. If need be, the measurement can also be effected after a drying of the test strips. The test strips can also be archived and repeatedly measured. The specific execution of the presently described, at least semi-quantitative test is apparent to the foodstuff chemist familiar with thin-layer chromatography. For example, the polarity of the test strip material can be adapted to the particular task. For example, use is thus made of pure nitrocellulose, or waxed or otherwise modified nitrocellulose (for instance blocked with a certain protein, e.g. BSA).The latter can be quite efficient to achieve better flow of the indicator dye.

Hereinafter, the reference signs will be elucidated and the figures will be more particulary elucidated together with said reference signs.

### List of reference signs

- 1: Mesoporous SiO₂
- 2: Aptamer, receptor protein, antibody or antibody fragment, for example Fab (antigen-binding fragment);
- 3: Organic molecule, hapten analogue;
- 4: Indicator substance: detection reagent, dye, SERS reporter, redox active substance;
- 5: Foodstuff contaminant, analyte;
- 10: Principle diagram of the course of events for an analysis, viewed microscopically;
- 15: Ready-to-use particle-based hybrid sensor material = mesoporous material 1, the indicator-filled pores of which are closed, e.g., by a hapten-analogue-held antibody;
- 15a-c: Ready-to-use particle-based hybrid sensor materials, all consisting of mesoporous material 1, the pores of which are, however, filled with different indicators and closed by antibodies held at different hapten analogues;
- 16: Particle-based hybrid sensor material after interaction of the antibody 2 with the analyte 5. The indicator substance, which was enclosed in pores of the SiO₂ as long as the antibody 2 which binds specifically to the analyte closed the pores, is released by interaction of the analyte with the antibody;
- 20: Principle diagram of the course of events for an analysis of a liquid foodstuff on the basis of 3 examples and one control, viewed macroscopically;
- 21: Test strip which is dipped into a liquid foodstuff 23. The content of particular analytes is to be tested.
- 22: Reservoir of the ready-to-use particle-based sensor material 15 (spot) on the test strip;
- 22a: Reservoir (spot) of the ready-to-use particle-based sensor material 15 on the test strip after a liquid foodstuff free of analytes, such as milk for example, has soaked the test strip (e.g. paper, nitrocellulose, glass fiber, nonwoven) and has risen in the test strip with the formation of a front 29. The rise is achieved according to the principles of paper or thin-layer chromatography. In the course of this, the liquid foodstuff flowing laterally across the reservoir wets the antibodies or antibody fragments bound only loosely (disengageably) to the pores of the mesoporous SiO₂. Since the liquid foodstuff did not contain any analyte to which the antibodies would have been able to bind, all the pores of the mesoporous material remain closed. The indicator substance present in the pores therefore cannot escape and reach the flow of the liquid foodstuff rising by capillary means. Accordingly, there is no occurrence either of any colouring of the test strip whatsoever. During any kind of scan of the test strip along its longitudinal direction, it will thus not be possible to capture a signal; at most, it will be possible to capture the "noise" occurring for the particular liquid foodstuff sample in interaction with the test strip.
- 23: Liquid foodstuff, in a sample container (left), already partly absorbed by the test strip by means of capillary forces and risen in the test strip with the formation of a progressing front 29 (right);
- 24: Test strip, the liquid foodstuff sample does not contain any analytes (control sample);
- 24a: The same test strip, the liquid foodstuff sample (control sample) did not contain any analyte; accordingly, all those mesoporous SiO₂ particles in the reservoir remain unchanged with respect to their starting state, which particles were closed by antibodies or antibody fragments which would have bound to a potential analyte - if said analyte would have been present at least in a minimum quantity in the examined liquid foodstuff.
- 25: Test strip, the liquid foodstuff sample contains the antibiotic A, which is detectable by release of the indicator specifically intended for the analyte "antibiotic A". The indicator can be detectable in an analytical manner (qualitatively and/or at least semi-quantitatively). Here, the measurement signal can be electrochemically capturable and/or fluorescently capturable and/or a Raman signal of a metal colloid (e.g. silver nanoparticles) can be detectable in an amplified manner as a SERS signal and/or a signal can be detectable in an amplified manner in another way. Here, SERS stands for surface-enhanced RAMAN scattering. However, this list is not conclusive. It is equally possible for the indicator to be a chemiluminescent substance. Also equally possible is a molecular probe suitable for highly sensitive detection with another spectroscopic method (e.g. SERS detection with metal nanoparticles). Self-evidently, it is possible for indicator substances having specific excitation and/or emission spectra in each case to be used in parallel to one another in a mix of different mesoporous SiO₂ particles filled with the indicator substances in each case, as a mixture in a reservoir on the test strip. These possibilities are depicted diagrammatically in Fig. 2.
- 25a: Test strip, the liquid foodstuff sample contained the antibiotic A; accordingly, particular pores of the reservoir were selectively opened and the indicator substance specifically assigned to the detection of the antibiotic A was released. The other pores, which are closed by other antibodies, remain closed. The reservoir is thus otherwise unchanged with respect to the starting state, apart from the fact that the indicator substance indicating the antibiotic A substantially completely escaped from, flowed out of or diffused out of the reservoir and/or was released therefrom.
- 26: Test strip, the liquid foodstuff sample contains the antibiotic B.
- 26a: Test strip, the liquid foodstuff sample contained the antibiotic B; accordingly, particular pores of the reservoir were selectively opened and the indicator substance specifically assigned to the detection of the antibiotic B was released. The other pores, which are closed by other antibodies, remain closed. The reservoir is thus otherwise unchanged with respect to the starting state, apart from the fact that the indicator substance indicating the antibiotic B substantially completely escaped from, flowed out of or diffused out of the reservoir and/or was released therefrom;
- 27: Test strip, the liquid foodstuff sample contains the mycotoxin A.
- 27a: Test strip, the liquid foodstuff sample contained the mycotoxin A; accordingly, particular pores of the reservoir were selectively opened and the indicator substance specifically assigned to the detection of the mycotoxin A was released. The other pores, which are closed by antibodies of other specificity, remain closed. The reservoir is thus otherwise unchanged with respect to the starting state, apart from the fact that the indicator substance indicating the mycotoxin A substantially completely escaped from, flowed out of or diffused out of the reservoir and/or was released therefrom.
- 28: Progression of the liquid foodstuff rising by means of capillary force, with the formation of the front 29;
- 29: Front of the liquid foodstuff 23 which rose by means of capillary force in the test strip 24, 25, 26, 27;
- 33: Specific release of that indicator which indicates the presence of antibiotic A in the liquid foodstuff;
- 33a: Chromatographically formed spot, comprising the indicator substance intended for the antibiotic A. Its position is, after completion of the test, situated immediately behind the front at the upper edge of the test strip subjected as a whole to flow-through by the liquid foodstuff (or one of the constituents thereof). In the terminology of a separation by means of thin-layer chromatography, said indicator substance has the largest Rf value. In this connection, an Rf value is commonly understood by experts to mean the relationship understood from the ratio of the path covered by the indicator substance in question proceeding from the starting point (the reservoir) to that altogether of the "mobile phase" - which corresponds here to the liquid foodstuff or one of its liquid constituents. Components migrating with or immediately behind the front have an Rf value close to 1; components (indicators) moving along only slightly from the application site (site of release, i.e. the reservoir) have an Rf value << 1.
- 34: Specific release of that indicator which indicates the presence of antibiotic B in the liquid foodstuff;
- 34a: Chromatographically formed spot, comprising the indicator substance intended for the antibiotic B. Its position is, after completion of the test, situated about halfway between the front at the upper edge of the test strip subjected as a whole to flow-through by the liquid foodstuff (or one of the constituents thereof) and the starting point (reservoir spot) on the test strip. In the terminology of a separation by means of thin-layer chromatography, said indicator substance has an Rf value close to 0.5.
- 35: Specific release of that indicator which indicates the presence of mycotoxin A in the liquid foodstuff;
- 35a: Chromatographically formed spot, comprising the indicator substance intended for the mycotoxin A. Its position is, after completion of the test, situated immediately after the reservoir on the test strip subjected to flow-through by the liquid foodstuff (or one of the constituents thereof). In the terminology of a separation by means of thin-layer chromatography, said indicator substance has the smallest Rf value (about Rf ∼0.1) and can thus be distinctly distinguished from the remaining indicator substances of a system.

The tables presented below list:
- Tab. 1:: Antibiotics and aflatoxins, the content of which in milk should be monitored, and the antibody-based tests suitable therefor.
- Tab. 2:: Fluorescent and chemiluminescent indicator substances which may be used for the suggested indicator reservoir.
- Tab. 3:: Examples concerning digestion deficiency causing substances.

In view of the suggested indicator reservoir, the suggested method, and the suggested use commercially available antibodies and aptamers specified by way of example in Tables 1 and 3 can be used for tailoring the suggested indicator reservoir for detection as described of, e.g., antibiotics, mycotoxins, and digestion deficiency causing substances.

**Table 1. Detailed list of antibiotics and aflatoxins which may be potentially found in milk**

| **Antibiotic class** | **% Total sold in EU**, **2012¹** | **Pharmacologically active substance** | **Commercial antibodies** | **Commercial aptamers** | **Maximum residue quantity in milk (µg l⁻¹)²** | **Commercial ELISA kits** |
|---|---|---|---|---|---|---|
| Tetracyclines | 36.9 | Tetracycline | Mono- or polyclonal (abcam ab30591) | Alphadiagnostic international AD-148-B; APTACAM. 18617415-Tetracyclines-6; Base-pair diagnostics Sigma, Cat# 87128 | 100 | MaxSignal Tetracycline ELISA test kit |
| | | Oxytetracycline | Polyclonal | | | |
| | | Chlortetracycline | - | | | |
| Penicillins | 22.3 | Benzylpenicillin | Polyclonal (abcam ab30592) | | 4 | |
| | | Amoxicillin | | | | MaxSignal amoxicillin ELISA test kit |
| | | Ampicillin | | Eurofins MWG Operon (AMP17) 5'-GCGGGC GGTTGTATAGCGG-3' | | Antibodies-online Creative diagnostics |
| | | Cloxacillin | | | 30 | |
| | | Oxacillin | | | | |
| | | Dicloxacillin | | | | |
| | | Nafcillin | - | | | |
| Sulphon-amides | 10.3 | Sulphadiazine | Poly- or monoclonal | | 100 (the total of all sulphon-amides) | MaxSignal sulphadiazine ELISA test kit |
| | | Sulphamethazine | Polyclonal (creative diagnostics (DPATB-H83239)) | | | MaxSignal sulphamethazine ELISA test kit |
| | | Sulphadoxine | Polyclonal | | | - |
| | | Sulphamethoxazole | Poly- or monoclonal | | | MaxSignal sulphamethoxazole ELISA test kit |
| | | Sulphamerazine | Polyclonal | | | |
| Macrolides | 8.0 | Erythromycin A | Polyclonal | | 40 | MaxSignal Erythromycin ELISA test kit |
| | | Spiramycin | Polyclonal (antibodies-online) | | 200 | MaxSignal Spiramycin ELISA test kit |
| | | Tilmicosin | Polyclonal (antibodies-online) | | 50 | MaxSignal Tilmicosin ELISA test kit |
| | | Tylosin | | | | MaxSignal Tylosin ELISA test kit |
| Polymyxins | 6.8 | Colistin | ELISA KIT (antibodies-online ABIN437922) | | 50 | MaxSignal colistin ELISA test kit |
| Aminoglycosides | 3.6 | Dihydro-streptomycin | Polyclonal (antibodies-online ABIN472584) | | 200 | |
| | | Streptomycin | Mono- or polyclonal (antibodies-online) | | | MaxSignal streptomycin ELISA test kit |
| | | Kanamycin A | Mono- or polyclonal (antibodies-online) | Eurofins MWG Operon (Ky2) 5'-TGGGGGTTGA GGCTAAGCCGA-3' | 150 | Ucbiodevices. Proximobiology Cusabio |
| | | Gentamycin | Mono- or polyclonal (antibodies-online) | | 100 | MaxSignal gentamycin ELISA test kit |
| | | Neomycin B | Mono- or polyclonal (antibodies-online) | | 1500 | MaxSignal Neomycin ELISA test kit |
| Lincosamides | 3.0 | Lincomycin | | | 150 | MaxSignal lincomycin ELISA test kit |
| | | Pirlimycin | Polyclonal (antibodies-online) | | 100 | - |
| Pleuro-mutilins | 2.9 | Tiamulin | ELISA KIT (almabion: alm29789) | | - | - |
| Fluoro-quinolones | 1.7 | Enrofloxacin | Mono- or polyclonal (antibodies-online) | | 100 | MaxSignal Enrofloxacin ELISA test kit |
| | | Danofloxacin | Polyclonal mesylated antibody (antibodies-online) | | 30 | - |
| | | Marbofloxacin | - | | 75 | - |
| | | Flumequine | Polyclonal (abcam ab59657) | | 50 | MaxSignal flumequine ELISA test kit |
| Diamino-pyrimidines | 1.6 | Trimethoprim | Polyclonal (antibodies-online) | | 50 | MaxSignal Trimethoprim ELISA test kit |

| **Aflatoxin class** | | | **Commercial antibodies** | **Commercial aptamers** | | **Commercial ELISA kits** |
|---|---|---|---|---|---|---|
| AFB1 | | | Mono- or polyclonal (antibodies-online) | | | MaxSignal AflatoxinB1 ELISA test kit |
| AFM1 | | | Mono- or polyclonal (antibodies-online) | Eurofins MWG Operon 5'-ACT GCT AGA GAT TTT CCA CAT-3' | | MaxSignal AflatoxinM1 ELISA test kit Aflasensor Test Kit (UNISENSOR) |

| | | | | | | |
|---|---|---|---|---|---|---|
| ¹ % Antibiotics for animals which produce foodstuffs or are themselves processed to form foodstuffs (in the EU, 24 countries, with the exception of Croatia, Greece, Malta and Romania) in 2012. (Sales of veterinary antimicrobial agents in 26 EU/EEA countries in 2012. 4th ESVAC Report, European Medicines Agency 2014.) ² Commission Regulation (EU) No. 37/2010, ec.europa.eu/health/files/mrl/mrl_20101212_consol.pdf 2010 | | | | | | |

**Table 2. Detailed list of indicator substances, applicable for the suggested indicator reservoir**

| ***Fluorescent substance*** | ***Chemiluminescent substance*** | ***Redox***-***active Substance*** |
|---|---|---|
| Coumarin and derivatives | Luminol and derivatives | Methylene blue |
| Rhodamine and derivatives | Lucigenin and other acridinium salts | Safranin T |
| Fluorescein and derivatives | Lophine | Safranin O |
| Tris(bipyridine)ruthenium(II) chloride and derivatives | | Diphenylamine |
| Sulphorhodamine B and derivatives | Oxalatest | 1,1'-Dibenzyl-4,4'-bipyridinium dichloride |
| Sulphorhodamine G and derivatives | Bis[4-(azidomethyl)phenyl] derivatives | Sodium diphenylamine-4-sulfonate |
| Sulphorhodamine 101 and derivatives | 9,10-Bis(phenylethynyl)anthracene | Tetrahydroxyquinone |
| BODIPY dyes and derivatives | 5,12-Bis(phenylethynyl)naphthacene | N,N'-Diphenylbenzidine |
| Naphthalimide and benzoxazole derivatives | 4,4'-Bis(1,2,2-triphenylvinyl)-1,1'-biphenyl | 1,5-Diphenylcarbazide |
| Styryl dyes | [(1,2-Diphenylethene-1,2-diyl)bis(4,1-phenylene)]diboronic acid | Methyl Orange |
| Pyrromethane dyes | 2,4,5-Triphenylimidazole | Methyl Red |
| Pyridine derivatives | Lucifer Yellow VS dilithium salt | α-Naphthoflavone |
| Oxazine derivatives | 4,4',4",4"'-(Ethene-1,1,2,2-tetrayl)tetraphenol | Neutral Red |
| | 1-{4-[1,2-Diphenyl-2-(*p*-tolyl)vinyl]phenyl}-1*H*-pyrrole-2,5-dione | Phenosafranin Dye |
| | 4,4'-(1,2-Diphenylethene-1,2-diyl)dibenzoic acid | Quinoline Yellow |

**Table 3. List of digestion deficiency causing substances**

| Digestive enzymes | Commercial antibodies | Commercial ELISA kits |
|---|---|---|
| Protease (serine 23) PRSS23 | Poly and monoclonal (Antibodies online; ABIN2785271; ABIN2665263 | Antibodies online, ABIN1565937 |
| Amylase | Poly and monoclonal (Antibodies online; ABIN411411; ABIN1857978 | Antibodies online, ABIN612663 |
| Lipase | Polyclonal (Antibodies online; ABIN2779368) | Antibodies online PNLIP (pancreatic lipase) ELISA Kit ABIN1884451 |
| Sucrase Isomaltase | Polyclonal (Antibodies online; ABIN2712095) | Cloud-Clone Corp. (SED186Hu) |
| Cellulase | Polyclonal (Antibodies online; ABIN890033) | Antibodies online ABIN2704071 |
| Lactase | Poly and monoclonal (Antibodies online; ABIN2782027; ABIN561652) | Antibodies online ABIN1450261 |

| Regulatory digestive hormones | Commercial antibodies | Commercial ELISA kits |
|---|---|---|
| Gastrin | Polyclonal (abbexa) https://www.abbexa.com/g astrin-antibody-1 | Raybiotech: RayBio^{®} Human/Mouse/Rat Gastrin EIA Kit Abcam (ab133033) |
| | | Proteintech Group Inc: Human GRP ELISA Kit |
| Secretin | Polyclonal (Antibodies online; ABIN2775496) | Raybiotech Human Secretin EIA Antibodies online (ABIN2538837) |
| cholecystokinin | Polyclonal (Antibodies online; ABIN2778154) | Antibodies online CCK ELISA Kit ABIN424292 |
| Motilin | Polyclonal (Antibodies online; ABIN113506) | Antibodies online ELISA Kit ABIN1116037 |

| | | |
|---|---|---|
| Substances causing digestion deficiency are for instance gluten, eggs, corn, peanuts and dairy due to the lack of the corresponding enzymes. Also, pathogenic organisms can affect digestion, for instance: Helicobacter pylori; Fungal dysbiosis/candidiasis (candida albicans is normally found in the digestive tract); or Small-bowel bacterial overgrowth (SBBO). | | |

As apparent, the fluorescence measured in a sample after exposure towards an antigen may be influenced by the contact time of the indicator reservoir with the analyte solution. In order to optimize both sensitivity and signal intensity different dyes and protocols for the preparation of indicator reservoirs have been experimentally tested.

**Fig. 4** shows the fluorescence enhancement of supernatants of an indicator reservoir prepared with lab-made hybrid mesoporous nanoparticles. The mesoporous MCM-41 nanoparticles were synthesized according to known procedures (Q. Cai, Z.-S. Luo, W.-Q. Pang, Y.-W. Fan, X.-H. Chen, F.-Z. Cui, Chem. Mater. 2001, 13, 258; S. Huh, J. W. Wiench, J.-C. Yoo M. Pruski, V. S.-Y. Lin, Chem. Mater. 2003, 15, 4247). Similar materials are commercially available as MCM-41, e.g. from Sigma-Aldrich, and can be used without further treatment.

The pores of the particles were filled with the dye sulforhodamine B (SRB). With the aim of loading the maximum amount of dye into the MCM-41 scaffold, a solution of the dye SRB sodium salt (2 mmol L⁻¹) was prepared in acetonitrile. Then, 2 portions of 12.0 mL were added to 300 mg of each MCM-41 nano- or microparticle material, yielding a suspension with a concentration of 0.8 mmol dye (g solid)⁻¹. The suspension was stirred for 24 h at room temperature. Two fractions of each type of material (2 x 40 mL) were centrifuged (5 min at 6000 rpm) and concentrated in a final volume of 1.5 mL. Thereafter, volumes of 0.14 mL and 0.7 mL of hapten solution (0.02 and 0.01 mmol) in DMF were added, arriving at final suspensions of 0.2 and 0.1 mmol (g solid)⁻¹. The resulting suspensions were stirred for 5.5 h at room temperature. Finally, the suspensions were centrifuged (5 min at 6000 rpm), and the solids obtained were washed with acetonitrile (0.75 mL), isolated by centrifugation, and dried at 35 °C in vacuum during 12 h. Another dye, e.g. BODIPY-PEG can be loaded into neat MCM-41 particles in the same manner as SRB.

The surface of the particles and thus the pore openings were modified with a sulfathiazole hapten, whereas an BSA-sulfathiazole conjugate was used as antigen for obtaining rabbit antisera against the hapten sulfathiazole (STZ) - an sulfonamide antibiotic.

The hapten was anchored after amidation reaction with 3-amino-propyl)triethoxysilane. In a first step, 0.1 mL of anhydrous DMF solutions of N-hydroxysuccinimide (NHS, 10 mg; 88 µmol) and N,N'-dicyclohexylcarbodiimide (DCC, 36 mg; 175 µmol) were prepared. Then each solution was added to a solution of the hapten (20 mg, 70 µmol) in anhydrous DMF (0.3 mL). The mixture was stirred at room temperature for 2 h and centrifuged. The mixture was stirred for another 4 h at room temperature and solids removed by centrifugation. In a second step, (3-aminopropyl)triethoxysilane (14.7 µL, 65 µmol) was added to the solution and the reaction mixture was stirred for 20 h at room temperature. The solution was centrifuged, and after the product formed it was left in 0.5 mL of DMF. Formation of the active ester intermediate and hapten derivative was confirmed by UPLC-MS. The hapten derivative was anchored to the surface of the mesoporous silica particles MCM-41. Finally, the particles were incubated with the polyclonal sera to obtain the corresponding antibody-gated mesoporous materials 15 (see **Figs. 1****,** **3**). To avoid unwanted leaching, a two-step capping procedure was followed: first, an incubation was carried out only with serum for 30 min. In a second step, BSA was added and the mixture incubated for another 1.5 h. For this purpose, fractions of 1 mg of the mesoporous materials (laboratory sample "M4") were suspended in 450 µL of physiologically buffered saline (PBS) containing 200 ppm of SRB and 2 µL of the serum and stirred for 30 min. Subsequently, 50 µL of a BSA solution (5%) were added to the suspensions, and left stirring for another 1.5 h. The material was centrifuged, washed 7 times with 1 mL PBS, dried 1 h in the vacuum at room temperature and stored in the fridge, yielding a material which was named as M4-A1@B.

In particular, **Fig. 4** shows the fluorescence which was registered at 585 nm (λ_{exc} = 560 nm) in presence (red; "2") and in absence (black; "1 ") of the analyte STZ (894 ppb) as a function of time for suspensions of this material M4-A1@B(N = 1) in PBS.

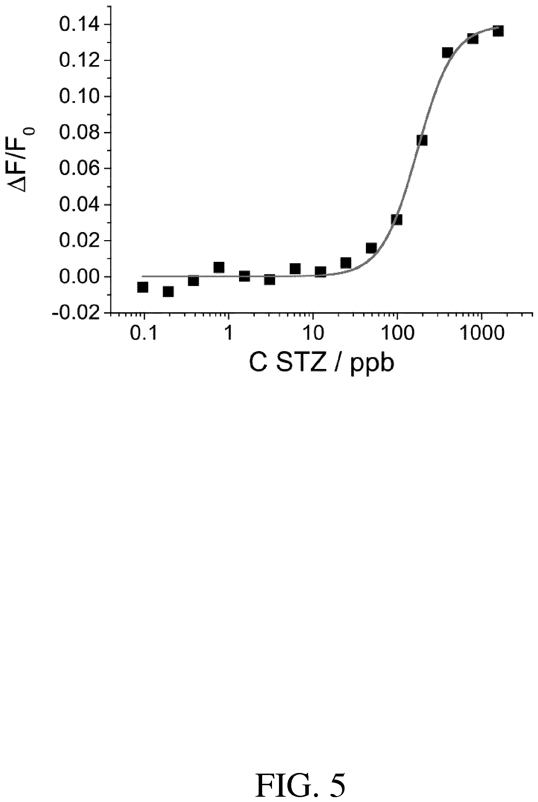

The fluorescence enhancement caused by STZ at different concentrations was assessed in milk as well. For these experiments, stock solutions of 100 µM STZ were prepared in milk. 600 µg of M4-A1 @B were suspended in 3300 µL of PBS and the suspension was divided into aliquots of 330 µL. Before adding STZ, 165 µL were taken from each aliquot to measure F₀ as before. Then, 6 µL of STZ solutions of different concentrations were added to the other fractions, the vials shaken by hand for 20 s and centrifuged before measuring the fluorescence of the supernatants in a fluorometer (λₑₘ = 585 nm, λ_{exc} = 560 nm). The obtained fluorescence signals are indicated in **Fig. 5****.** The results suggest that the dynamic range is largely similar as that in PBS buffer. By fitting the curve to a logistic function it is possible to derive an IC20, i.e. a limit of quantification (LOQ), of 87 ± 8 ppb and an IC50 of 172 ± 12 ppb.

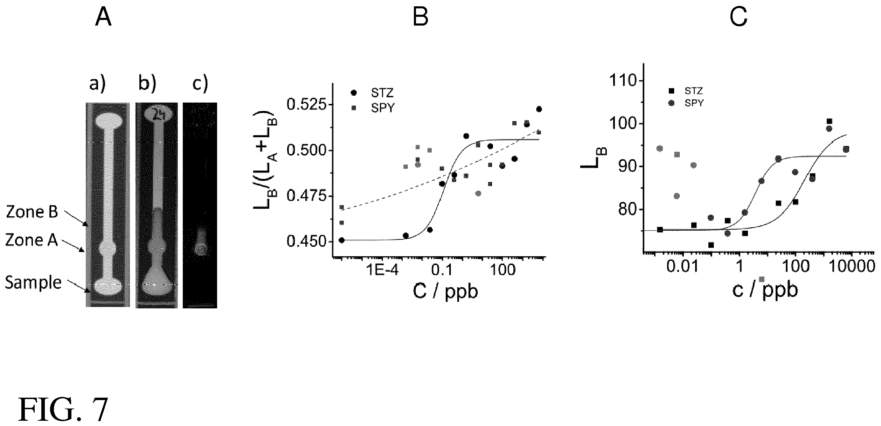

Encouraged by these results, the gated indication system was further tested for possible use with test strips in the sense of a rapid and simple lateral flow-type of test with fluorescence readout. As explained above, after development the strips show two different zones, a first one (zone A) at which the sensing material (indicator reservoir) has been deposited, and a second one (zone B) in which the released dye is collected that could travel with the solvent front (see **Figs. 2****,** **7**).

In case of absence of a sulfonamide, the second zone (zone B in **Fig. 7**) should show minimum signal. In case of presence, the fluorescence in that zone should correlate with analyte concentration. The amount of dye released can be quantified with a fluorescence reader with appropriate excitation and emission wavelengths or by taking pictures under proper light excitation conditions. Strips were prepared using a Hi-Flow nitrocellulose paper from Millipore^{®}. Strips of 0.5x5cm were cut and 2 µL of a suspension of M4-A1 @B (5 mg mL⁻¹) in PBS were spotted at the first zone (Zone A) (300 µg in 60 µL of PBS). Strips were left at room temperature for drying.

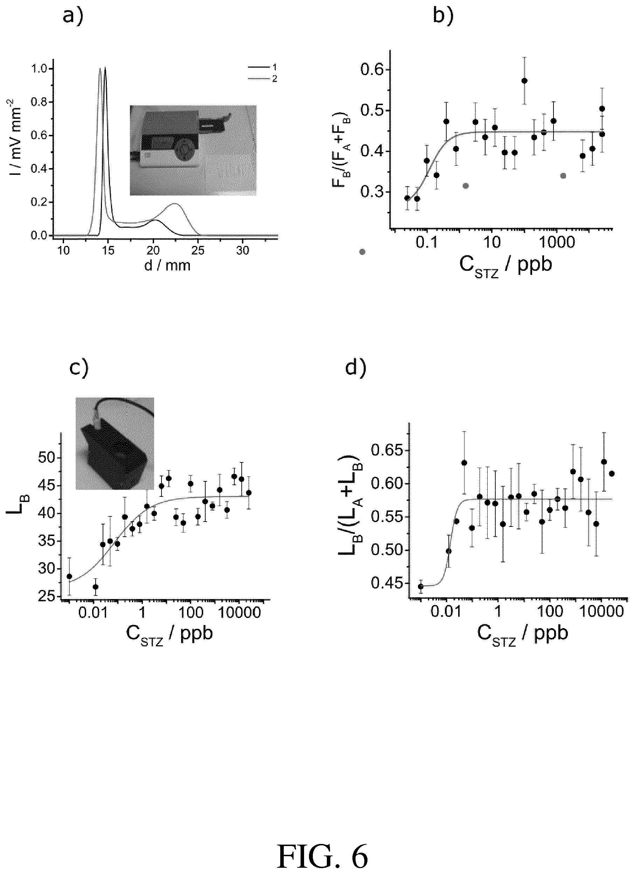

Afterwards, the strips were dipped into 150 µL of raw milk spiked with various amounts of STZ, developed and let dried at room temperature. The experiment was performed 2 times, one with milk, which was stored for 6 d in the fridge and the other one with fresh milk from a small farm, stored for 1 d. Afterwards, the fluorescence histograms were registered with a lateral flow reader and a home-made housing equipped with an LED (λ_{exc} 505 nm) as excitation source and filters (532 nm cut-off and 550 nm long-pass) that can be fitted to a conventional digital camera. The results are shown in **Fig. 6. Fig. 6** **a**) depicts typical histograms recorded with the reader, revealing the increase in fluorescence in the second zone in the presence of STZ. Analysis of an assay run as presented in **Fig. 6** **b**) shows the ratio between the integral value of dye released in the second zone and the total dye signal (first and second zones). Using this approach, it is possible to achieve an IC50 below 1 ppb, yet because of the simple experimental setup the data are considerably noisy. **Fig. 6** **c**) (luminance of dye released in second zone and **Fig. 6** **d**) (ratio of luminance) allows to compare the reader results with those obtained by extracting the luminance from images taken with a camera. This parameter corresponds to the mean coordinate number of the histogram of the "Y" coordinate of the XYZ CIE 1931 color space of each spot. The data reported suggest that the uncertainty is higher for the used reader, presumably due to repositioning effects in the strip holder.

However, since interfering effects from the matrix as such cannot be excluded, we repeated the experiments with milk centrifuged prior to conducting the assay and with letting the flow only run up to a fixed distance of 3 cm for all strips. The reproducibility was improved.

In particular, **Fig. 6** shows: a) Fluorescence histogram (λ_{exc} 560 nm; λₑₘ 625 nm) registered with the lateral flow reader for strips containing a spot of M4-A1 @B at ca. 15 mm after development in 150 µL of raw milk in absence (black) and presence (red) of 2 ppm of STZ. Inset: Photo of the lateral flow reader. b) Corresponding fluorescence enhancement of dye release (F_{B} /(F_{A} +F_{B})) of the signal of strips obtained with the reader as a function of concentration of STZ (λ_{exc} 565 nm, λₑₘ 625 nm). The red points were classified as outliers and not considered in the fit. c) Changes of luminance in B zone (L or "Y" value of the CIE 1931 XYZ color space) of the strips as a function of the concentration of STZ. Inset: photo of the home-made housing lamp. D) Corresponding luminance enhancement (L_{B} /(L_{A} +L_{B}) as a function of the concentration of STZ (N = 2).

With the aim to reduce the error of the measurements, sensing strips were prepared by patterning hydrophobic walls of wax in the nitrocellulose paper using a commercially available printer and leaving in an oven at 110°C during 1 min to melt the wax and to create the hydrophobic barriers across the thickness of the paper. The design of the hydrophobic barriers is depicted on **Fig. 7A****.**

Strips of 0.6x4 cm were cut and 2 µL of a suspension of M4-A1@B (5 mg mL⁻¹) in PBS were spotted at zone A following the same procedure as before. Thereafter, 40 µL of milk containing certain amounts of STZ (sulfathiazole) or SPY (sulfapyridine) were added to the "sample" zone, and milk was flown during ca. 3.5 min until the flow stopped. The excess of milk was removed from the sample zone, and the strip was left to dry at room temperature. Subsequently, measurements of the luminance were only performed with the home-made lamp (**Fig. 7**).

In particular, **Fig. 7** shows: A) Picture of the designed strips (a) before flow, (b) during flow and (c) dried, under excitation with home-made lamp. B) Ratio of luminance data registered with the camera as a function of STZ (black) and SPY (blue), N = 1. C) Corresponding data measured for the changes of luminance in zone B of the strips. The red points were classified as outliers and not considered in the fit. Of course, any other technology or method known to be applicable in lateral flow devices and/or assays could be applied instead of the strips which are shown here as a mere example illustrating the gist of the invention.

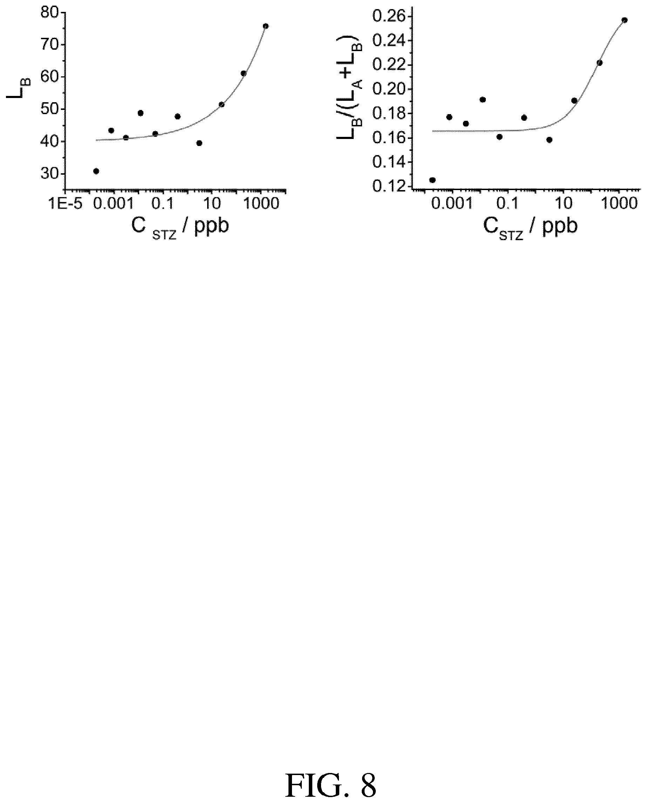

Further, to corroborate the effect of the milk on leaching behavior, experiments were performed in PBS instead with strips of 0.6x4 cm containing hydrophobic barriers as designed before. 2 µL of a suspension of M4-A1 @B (5 mg mL⁻¹) in PBS were spotted at zone A (cf. **Fig. 7**) following the same procedure as before. Afterwards, 40 µL of PBS containing several amounts of STZ were added to the "sample" zone and solvent was flown for 3.5 min. As can be seen in **Fig. 8****,** an enhancement of the luminance was observed as a function of the concentration, showing similar sensitivity to M4-A1@B in suspension.

In particular, **Fig. 8** shows (on the left) luminance data registered with a camera as a function of STZ-concentration flowing with PBS buffer and (on the right) corresponding ratio of luminance as a function of the concentration of STZ (N=1).

A biphasic extraction assay can significantly improve the performance of indicator release systems. Since the dye partitioning coefficient of sulforhodamine B (SRB) is too low to undergo efficient phase transfer from water/milk to CHCl₃ we relied on a water-soluble PEGylated borondipyrromethene (BODIPY) dye. Capping of the material was performed following the same conditions as for the preparation of M4-A1 @B, but in this case 1 mg of the mesoporous silica was suspended in 450 µL of PBS containing 360 ppm of BODIPY-PEG and 2 µL of the serum for 30 min. Thereafter, 50 µL of BSA solution (5%) were added to the suspensions and stirred for another 1.5 h. The material was centrifuged and washed only 3 times with 1 mL PBS because this dye shows a much smaller tendency for leaching than SRB does. Before centrifuging the suspension in the last washing cycle, it was divided into 10 fractions, which were dried separately for 1 h in the vacuum at room temperature and stored in the fridge, yielding 10 fractions of ca. 100 µg of M7-A1@B.

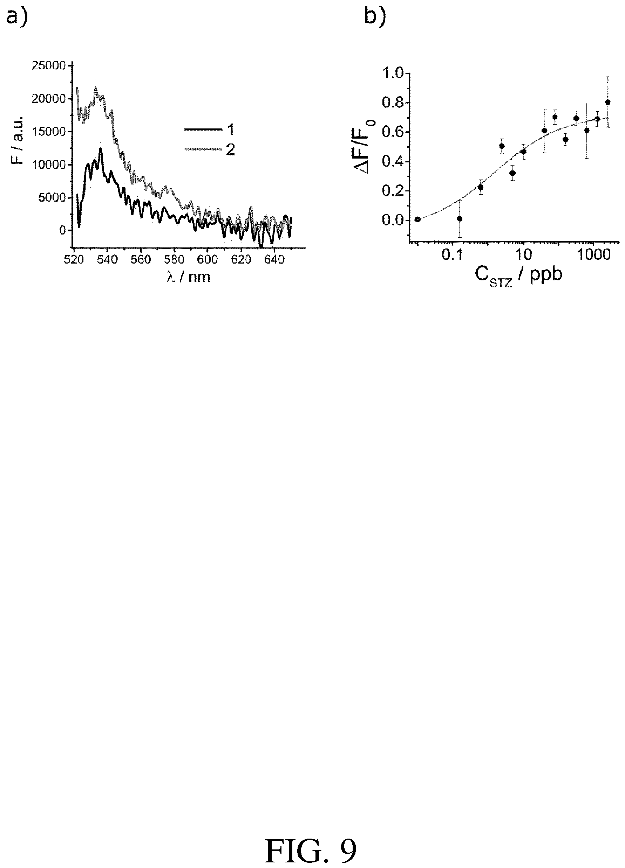

In an attempt to avoid the extraction process, and with the aim to compare the effect of different dyes to M4-A1 @B, respectively, first of all the material M7-A1 @B was tested in absence and in presence of several amounts of STZ following similar a procedure as explained previously. For that purpose, two independent experiments were performed, using in one case milk provided from FOSS stored in the fridge (6 d) and fresh milk from Poland (1 d in the fridge). 200 µg of material were suspended with 2 mL of milk, and the suspension was divided into aliquots of 150 µL. Before adding STZ, 70 µL were taken from each aliquot to measure F₀ as also described before. Then, 20 µL of STZ solutions of different concentrations were added to the rest of the fractions (80 µL), the vials were shaken in the thermomixer at 800 rpm for 10 min, centrifuged and the fluorescence of the supernatants measured at 535 nm with λ_{exc} = 515 nm (**Fig. 9**).

Despite the pronounced scattering observed, it was possible to trace the increasing signal of dye fluorescence after subtraction of the signal of the neat milk. **Fig. 9** reveals an improvement in sensitivity with respect to M4-A1 @B, due to a suppression of leaching of dye in the absence of STZ. By fitting the curve to a logistic function it was possible to derive a limit of quantification (LOQ) of 65 ± 18 ppt and an IC50 of 1.5 ± 1.4 ppb, which is ca. 2 orders of magnitude more sensitive than M4-A1@B.

In particular, **Fig. 9** shows a) Fluorescence spectra of BODIPY-PEG released in milk suspensions containing M7-A1@B in absence (1; black) and in presence (2; red) of 2 ppm of STZ (λ_{exc} = 515 nm). b) Fluorescence enhancement of the supernatants registered at 535 nm (λ_{exc} = 515 nm) of suspensions of M7-A1@B as a function of the concentration of STZ added after 10 min. of interaction time; (N = 2).

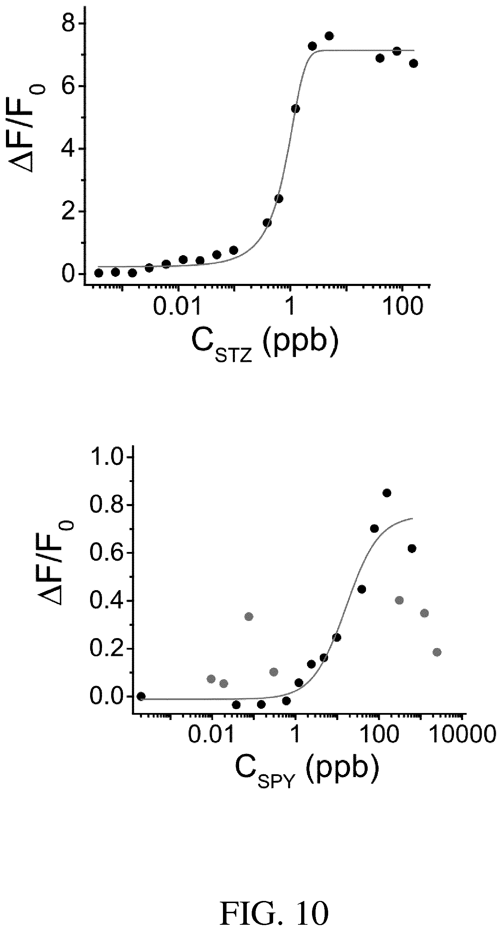

Next, the effect of the concentration of STZ and SPY was studied. For this purpose, we repeated the procedure but added different concentrations of the corresponding sulfonamide into each fraction and took an aliquot from the chloroform phase after 10 min of reaction (**Figure 10**). In this case, milk stored in the fridge for 5 and 7 d was used. It is obvious that a highly sensitive response could be found in both cases, with an LOQ of 0.5 ± 0.1 ppb and an IC50 of 0.8 ± 0.1 ppb for STZ. For SPY, both the fluorescence increase and the sensitivity are lower (LOQ: 4.6 ± 2.2 ppb, IC50: 16.2 ± 6.9 ppb).

In particular, **Fig. 10** shows the fluorescence increase of the CHCl₃ phase at 538 nm (λ_{exc} = 515 nm) after dye extraction from suspensions of M7-A1@B in raw milk as a function of STZ (top) and SPY (bottom) concentration after 10 min reaction time. The red points were classified as outliers and not considered in the fit (N = 2, only one repetition shown).

The sensitivity could further be improved by increasing the number of washing cycles of the material before use, although the fluorescence enhancement is lower due to partial dye removal during continued washing. In particular, LOQ and IC50 values are improved (LOQs of 0.02 ± 0.01 ppb for STZ and 0.78 ± 0.38 ppb for SPY and IC50 of 0.28 ± 0.15 ppb for STZ and 4.4 ± 1.7 ppb for SPY), which might be due to better suppression of blank release. Furthermore, the amount of dye released in the working range of the assay at lower STZ concentrations (100 ppt) is at least 100 times higher, preferably 200-times higher than that of antibody displaced, decreasing to 80-times at concentrations higher than 50 ppb, which is a remarkable sign of signal amplification especially in the desired low-concentration range.

Exemplary embodiments of the presently described materials and methods are thus worded briefly and concisely:
1. A hybrid mesoporous nanoparticle or microparticle material which contains a chemical indicator substance within the pores and in which a particular organic molecule (hapten) is covalently bonded on the outer surface, which molecule has an affinity for a biological molecule (e.g. antibody, receptor protein or aptamer) and is structurally similar to the target substance (e.g. an antibiotic, a mycotoxin, e.g. aflatoxin, or a hormonally active substance).
2. The hybrid mesoporous nanoparticle or microparticle material according to point 1, wherein periodic mesoporous silica having mesopores between 2-50 nm and specific surfaces of from 500 up to 1500 m²g⁻¹ are used (e.g. MCM-41, HMS, MSU-n, MSU-V, FSM-16, KSW-2, SBA-n (n = 1, 2, 3, 8, 11-16), UVM-7, UVM-8, M-UVM-7 or M-UVM-8)(e.g. Sigma-Aldrich). The mesoporous silica material referred to can also contain, via the synthesis of the same, integrated magnetic particles (e.g. Fe₂O₃).
3. The hybrid mesoporous nanoparticle or microparticle material according to point 1, wherein a coloured, fluorescent or chemiluminescent dye (see Table 2) is contained in the pores as optical or electrochemical indicator substance.
4. The hybrid mesoporous nanoparticle or microparticle material according to point 1, wherein an organic molecule similar to the target substance (e.g. antibiotic or mycotoxin, see Table 1) is covalently bonded to the silica support particle. The molecule can contain either an alkoxysilane group for reacting with the silanol groups of the mesoporous material, or be directly fixed on the surface via a functional group immobilized on the mesoporous material (e.g. amino or carboxylic acid group). The material can additionally bear on the surface other organic molecules which, for example, can further increase the specificity or reduce non-specific bonds (e.g. poly(ethylene oxide)).
5. The hybrid mesoporous nanoparticle or microparticle material according to point 1, wherein a commercially available or self-produced biomolecule (e.g. antibody or aptamer) is used for closing the pores, which biomolecule has a high affinity for the target substance and a somewhat low affinity for the hapten.
6. A method according to any of points 1-5, wherein, for example, 1 µl of materials in a 5 mg ml⁻¹ concentration can be spotted or striped, deposited by any suitable method, onto nitrocellulose strips, nitrocellulose having a partial wax coating, or some other support material, e.g. glass fiber mesh fabric or glass fiber paper.
7. The method according to point 6 or to any of points 1 to 6, wherein the released indicator substance can be detected either via a fluorescence reading device, a luminescence reading device or a camera (e.g. mobile communication device, digital camera) in a qualitative and at least semi-quantitative manner, if not in a quantitative manner.

Although specific embodiments have been depicted and described herein, it is within the scope of the present invention to appropriately modify the shown embodiments without deviating from the scope of protection of the present invention. The following claims represent a first, non-binding attempt to generally define the invention.

## Claims

1. An indicator reservoir, comprising a porous support material having pores and an indicator substance which is contained in the pores of the porous support material, wherein the pores of the porous support material are closed by a pore-closing material forming non-covalent bonds with the porous support material, wherein the pore-closing material is selected to specifically bind an analyte in a liquid selected from: a liquid foodstuff, a liquid agricultural sample and a liquid used to extract the analyte from a non-liquid foodstuff or agricultural product; and wherein the indicator substance is released from unclosed pores, when the analyte specifically binds to the pore closing material and the non-covalent bonds are disengaged.

2. The indicator reservoir according to claim 1, wherein the pore-closing material is non-covalently bound to the porous support material by an compound that is anchored to the porous support material, wherein the compound that is anchored to the porous support material comprises a hapten, an organic molecule, a complex of a metal with an organic ligand, a nucleic acid, a strand fragment of a nucleic acid, or any epitope or peptide molecule and/or wherein wherein the pore-closing material comprises an antibody, an antibody fragment, a receptor protein, or an aptamer.

3. The indicator reservoir according to at least one of the previous claims, wherein the pore-closing material is adapted to specifically bind an analyte substance or a specific group of analyte substances selected from: a toxin, an antibiotic, a hormone or a hormonally-effective substance, an allergen, a digestion deficiency causing substance, transmissible spongiforms, a genetically modified organism or constituent thereof, an adulterant, a nutrition additive, or an enzyme, and/or wherein the pore-closing material is adapted to specifically bind to an analyte micro-organism or a specific group of analyte micro-organisms selected from: a bacteria, a bacteria spore, a pathogen, a mold, a yeast, a protozoon, a zoonosis causing agent, or a virus, and/or wherein the pore-closing material is adapted to specifically bind to an analyte that is a specific sequence or a group of specific sequences of nucleotides.

4. The indicator reservoir according to either of the preceding claims, wherein the pore-closing material has a higher affinity with respect to the analyte than with respect to the compound, that binds it to the support material.

5. The indicator reservoir according to any of the preceding claims, wherein the porous support material comprises a mesoporous material, wherein the mesoporous material comprises at least one of silica and alumina particles.

6. The indicator reservoir according to claim 5, wherein the particle comprises a magnetic particle or a paramagnetic particle.

7. The indicator reservoir according to any of the preceding claims, wherein the indicator substance is selected from a fluorescent material, from a chemiluminescent material, from an electrochemically active substance and/or from a material having a specific RAMAN spectrum or a specific IR spectrum.

8. The indicator reservoir according to any of the preceding claims, wherein the compound that is anchored to the support material is covalently or non-covalently bonded or adsorbed to the support material.

9. The indicator reservoir according to any of the preceding claims, wherein the support material comprises an organic molecule that increases the specificity of the pore-closing material with respect to the analyte or reduces a non-specific bond with respect to said analyte, and/or wherein the support material
- is a periodic mesoporous silica having mesopores between 2-500 nm, for example between 2-20 nm, more particularly between 2-5 nm; and
- has a specific surface area within a range selected from 400 to 2000 m²g⁻¹, 500 up to 1500 m²g⁻¹, particularly within the range between 600 up to 1200 m²g⁻¹.

10. The indicator reservoir according to at least one of the previous claims, wherein the liquid is a liquid foodstuff selected from: a water, a milk, a wine, a non-alcoholic beverage, a beer, a cider, a honey, an egg or a constituent thereof, an edible oil, or a waste water from the food industry;
and/or
wherein the liquid is a liquid agricultural sample selected from a water used for agricultural irrigation or for processing of a harvested crop, or a waste water including water coming from drainage of animal farms and/or of cultivated soils, a physiological fluid that is taken from livestock animals to monitor their health and well- being, selected from a blood, a blood component, an urine or an extracellular fluid sample;
and/or
wherein the liquid is a liquid that was used to extract the analyte from non-liquid substances selected from a grain, a rice, a corn maize, a coffee bean and/or a seed of all kinds, a meat, a fish, a sea food, a plant or vegetable that are used as food and/or animal feed, a milk powder, a non-liquid milk product, a flour, and/or a product of a milling process.

11. The indicator reservoir according to at least one of the previous claims, wherein the analyte originates from a fertilizer; a pesticide such as a fungicide, an acaricide, an insecticide, an herbicide, a rodenticide or a growth regulator; a seed treatment or a seed dressing material.

12. The indicator reservoir according to any of the preceding claims, wherein the indicator reservoir is arranged on a test strip, so that a liquid wetting the test strip releases the indicator substance from the pores of the indicator reservoir once an analyte contained in the liquid is bound by the pore-closing material owing to the affinity thereof, and an disengageable bond in relation to the compound is disengaged, and, as a result, the indicator substance is released from the pores of the porous support material.

13. A system for monitoring an analyte in a liquid foodstuff, in a liquid agricultural sample, or in a liquid extract from a foodstuff or from an agriculture-related substance, comprising:
the indicator reservoir according to any of the preceding claims,
a wetting means for wetting or mixing the said reservoir with the said liquid in order to allow the release of said indicator substance in accordance with a concentration of the analyte in the liquid,
a separating means to separate the indicator reservoir from the liquid containing the released indicator, and
a detecting means to detect and/or to quantify a concentration of the released indicator.

14. The system of claim 13, comprising a plurality of different indicator reservoirs, each of the pluralities allowing the detection of a different analyte.

15. The system according to claim 13 or 14, wherein a difference among different indicator reservoirs lies in a spectral, an electrochemical and/or a plasmonic resonance characteristic of the indicator substance.

16. The system of any of claims 13 to 15, wherein
- for an optical detection the detecting means comprise an illumination source and an optical detector that are coupled to an optical filter array and a spectrophotometer, optionally incorporating waveguides or optical fibres;
- for an electrochemical detection the detecting means comprise a galvanometer and/or a potentiostat which are connected to electrodes; and
- for an opto-electrochemical detection the detecting means comprise a galvanometer and/or a potentiostat which are connected to electrodes and also coupled to an optical detector;
and/or
wherein the separating means for separating the indicator reservoir from the liquid containing the released indicator is selected from: a magnet; a centrifuge; a membrane filter; a lateral-flow strip; a liquid phase extraction module; a micro-fluidics separation module; and/or a magnet and magnetic particles, the magnetic particles being functionalized with an agent that binds to the pore-closing material defined in claims 1 to 12.

17. The system according to claim 16 in which the agent with which the magnetic particles are functionalized comprises an antibody or an antibody fragment against the pore-closing material, wherein optionally said antibody or antibody fragment has a lower affinity to the pore-closing material than the pore-closing material has to its compound that is binding it to the porous support material and the affinity that the pore-closing material has to the analyte.

18. A test strip adapted for a "lateral flow" assay, comprising at least one segment comprising an indicator reservoir according to any of claims 1 to 12.

19. The test strip according to claim 18, further comprising at least one segment modified by a metal colloid and/or by a metallic nanoparticle.

20. The test strip according to claim 18 or 19, wherein the test strip comprises a nitrocellulose, a paper, or a glass fiber mesh fabric.

21. An analysis method for detecting an analyte in a liquid foodstuff, a liquid agricultural sample, or a liquid that was used to extract the analyte from other type of foodstuff or agriculture-related substance, comprising:
- providing an indicator reservoir, as claimed in any of the claims 1 to 12;
- wetting the indicator reservoir with a liquid containing the analyte;
- determining a release of the indicator substance, the determination being effected in a qualitative and/or at least semi-quantitative manner.

22. The analysis method according to claim 21, wherein the wetting is achieved with a formation of a directed flow of a liquid containing the analyte and with at least a partial formation of a front of the liquid or a constituent thereof on the test strip, so that by means of a position of the released indicator substance on the test strip, it is possible to unambiguously determine the presence of the analyte and/or to quantify it with the aid of a reading device, wherein the reading device is selected from a group, comprising: a single-handedly operable reading device or scanner, a telecommunications terminal device, a mobile telephone or "smart phone", a digital camera.
